(19) Europäisches Patentamt
European Patent Office
Office européen des brevets

(11) Publication number: 0 245 859 B1

(12) EUROPEAN PATENT SPECIFICATION

(45) Date of publication of patent specification: 17.10.90

(51) Int. Cl.[5]: C 07 C 39/367, C 07 C 37/62

(21) Application number: 87106988.6

(22) Date of filing: 14.05.87

# (54) Process for the preparation of brominated bisphenols.

(30) Priority: 16.05.86 US 864065

(43) Date of publication of application:
19.11.87 Bulletin 87/47

(45) Publication of the grant of the patent:
17.10.90 Bulletin 90/42

(84) Designated Contracting States:
AT BE CH DE ES FR GB IT LI NL SE

(56) References cited:
EP-A-0 214 722
GB-A-1 278 603
US-A-3 720 721
US-A-3 894 094
US-A-3 929 908
US-A-3 956 403
US-A-4 058 570

JUPAC (Edition 1979) Nomenclature C-71.4;
Russian Chemical Reviews, Vol. 32, No. 2, pages 79-82, 1963; Journal Chemical Society Perkin II, 933 (1979)

(73) Proprietor: THE DOW CHEMICAL COMPANY
2030 Dow Center Abbott Road P.O. Box 1967
Midland Michigan 48640-1967 (US)

(72) Inventor: Mendoza, Abel
3001 Gibson Street
Midland, Mich. 48640, (US)

(74) Representative: Sternagel, Hans-Günther, Dr. et al
Patentanwälte Dr. Michael Hann Dr. H.-G. Sternagel Sander Aue 30
D-5060 Bergisch Gladbach 2 (DE)

EP 0 245 859 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to the formation of brominated biphenols by direct bromination.

Brominated biphenols are reaction intermediates useful in the formation of flame-retardant polymers and monomers. Biphenols have long been used as reaction intermediates in the formation of polymers and monomers. The incorporation of bromine improves the flame-retarding properties of the compositions which incorporate the biphenol.

In biphenols there are two types of ring hydrogen atoms: those ortho from one of the phenolic moieties, that is, in any of the 2, 2′, 6 or 6′ positions and those meta from one of the phenolic moieties, that is, in any of the 3,3′,5,5′ positions. This is illustrated in the following formula:

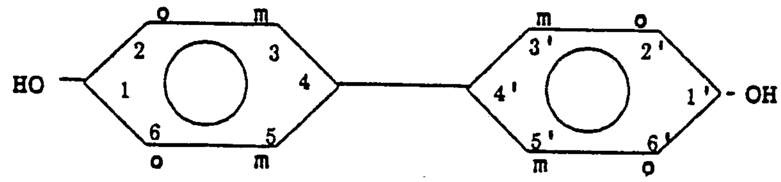

The ortho position is the position where bromination of unsubstituted biphenol occurs. Bromine atoms in the ortho position are less stable than bromine atoms in the meta position.

Meta-brominated biphenols have been prepared by reacting bromine and diphenoquinones. This reaction is extremely exothermic, requiring either slow addition of reactants such as described in US Patent 3,956,403 or cooling of the reaction to low temperatures such as described in US—A—4,058,570, where yields of 71 percent to 75 percent are obtained.

Meta-halogenated biphenols have also been prepared by reacting diphenoquinones with a hydrogen halide, as described in US—A—3,720,721; 3,748,303; and 3,894,094. However, this approach is limited to the introduction of two halogens. Additionally, as recognized by US—A—3,720,721, contacting a tetramethylbiphenol with elemental chlorine leads to a mixture of products including products having the organo substituent of the biphenol halogenated.

The bromination of biphenol in the absence of solvent to produce octabromobiphenol is generically described in GB—A—1,278,603. However, in this system, benzylic substitution is not a possible concern since the biphenol does not have the ortho positions blocked.

It would be desirable to have a selective process for the preparation of meta-brominated biphenols without the problems incident to the high exothermic reaction. Further, it would be desirable to have a process which could produce meta-brominated biphenols in improved yields, which would give substantially no benzylic bromination, and which would be industrially more convenient.

The present invention is a process for preparing a meta-brominated biphenol having at least 2 meta-bromine atoms and having the formula

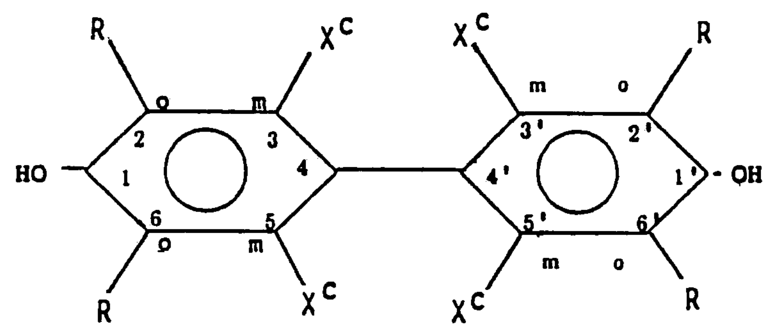

wherein R is a $C_1$—$C_8$ alkyl or alkoxy group, X is Br or hydrogen characterized in that a starting compound of the formula

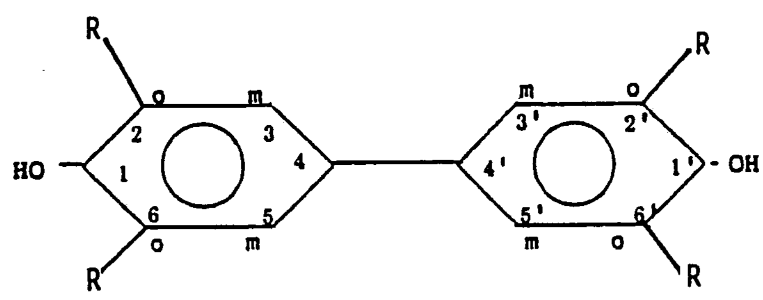

having all its ortho positions blocked with a $C_1$—$C_8$ alkyl or alkoxy group R, is contacted with a brominating agent selected from bromine, bromine chloride and hypobromites at a temperature range from 0 to 100°C.

It is surprising that the blocked bisphenol is readily brominated in the meta position by the process of the present invention to give a high yield of the desired product. These meta-brominated biphenols are chemical intermediates useful, e.g., in the formation of flame-retardant monomers and polymers. It is also surprising that in products wherein the blocking radical is bound to one of the biphenol's benzene rings with a carbon atom, the product has no benzyl bromides detectable by nuclear magnetic resonance.

For the purposes of this invention, a bonding position is blocked when it contains a moiety which does not cleave upon contact with the brominating agent and is a $C_{1-8}$ alkyl or alkoxy moiety. Preferred blocking moieties are alkyl or alkoxy radicals, such as methyl, cyclohexyl, methoxy and ethoxy radicals. The most preferred blocking moiety is methyl. Primary alkyl moieties are preferred.

For the purposes of this invention, a brominating agent contains or generates bromine in a manner sufficient to meta-brominate the biphenol.

The brominating agents are bromine, bromine chloride and hypobromites. Elemental bromine is more preferred.

Biphenols which have all of the ortho positions blocked can be prepared, for example, by the method described in *European Polymer Journal,* Vol. 6, pp. 1339—1346 (1970). Preferred are biphenols in which each blocking moiety is the same. More preferred biphenols are 2,2′,6,6′-tetramethylbiphenol, 2,2′,6,6′-tetraethylbiphenol or 2,2′-dimethyl-6,6′-diethylbiphenol. The most preferred biphenol is 2,2′,6,6′-tetramethylbiphenol.

The reactants may be contacted in any order and amount. It is preferred to use a 25 pecent excess of the brominating agent when a catalyst is present and a twofold excess of the brominating agent when no catalyst is present. The contacting can be conducted in any container which contains the reacting mixture. The process can be conducted at any combination of temperature and pressure at which the bromination reaction will take place, the reaction temperature being of from 0°C to 100°C, preferably at temperature of from 0°C up to the reflux temperature of the reaction mixture or higher. More preferably, the temperature is from 20°C to 50°C. Most preferably, the reaction is carried out at the reflux temperature of the reaction mixture. Typically, the reaction is carried out at atmospheric or elevated pressures. Preferably, ambient pressure is employed.

The contact is maintained until the desired conversion of reactants occurs, typically four hours. Typically, conversions of the biphenol are greater than 80 percent. Preferably, the biphenol conversion is above 90 percent, more preferably above 95 percent and most preferably above 99 percent.

As the reaction typically generates hydrogen bromide, the reaction container is preferably closed and swept with an inert gas, such as nitrogen, into a water scrubber in order to recover the hydrogen bromide.

A bromination catalyst is optionally employed in the process of the present invention. Friedel-Crafts catalysts are preferred, and are well known. Examples of bromination catalysts include the halides of metals such as iron, aluminum and tin. Examples of preferred catalysts include aluminum bromide, aluminum chloride and iron chloride with iron chloride (ferric chloride) being most preferred. The catalyst is employed in catalytic quantities. Preferably, the amount of catalyst employed ranges from 0.1 to 5 weight percent of catalyst based on the weight of bisphenol compound employed.

A reaction medium advantageously is employed in the process of the present invention. The reaction medium functions to wholly or partially solubilize or suspend the reactants and reaction products, and to aid in heat transfer. While the amount of reaction medium employed can vary widely, the amount of reaction medium typically ranges from 8 to 25 moles of reaction medium per mole of biphenol. Preferably, from 15 to 20 moles of reaction medium are employed per mole of biphenol. Preferred reaction media include essentially nonreactive halogenated hydrocarbons, such as relatively low boiling halogenated aliphatic hydrocarbons, e.g., carbon tetrachloride, methylene chloride, chloroform, bromochloromethane, bromotrichloromethane, trichloroethane, and the like, as well as mixtures of these compounds. It is especially preferred to employ methylene chloride as the reaction medium.

When the contacting is performed under reaction conditions as specified hereinbefore, the resulting meta-brominated biphenol can be recovered by processing the resulting reaction mixture in a variety of known workup procedures to isolate the brominated products. The crude reaction mixture can, for instance, be subjected to stripping either at atmospheric pressure or, preferably, under reduced pressure to the point of constant weight of the residue. The crude product which is thus isolated can be further purified, for instance by recrystallization or by digestion with a recovery medium such as acetone, toluene, methanol or water.

The final yields typically are at least 70 mole percent based on the starting biphenol. Preferably, the yields are at least 75 mole percent, more preferably at least 80 mole percent and most preferably at least 90 mole percent. As used herein, the term "yield" is the numerical product of conversion and selectivity, based on product actually isolated. Conversion refers to the conversion of the starting material to any other material. Selectivity is the actually recovered molar amount of a given product expressed as a fraction or percent of the theoretically possible amount from the feed material converted.

The meta-brominated biphenol is typically brominated in all nonblocked meta positions. However, the product can be a mixture of fully meta-brominated biphenols and biphenols which have some hydrogen in the meta positions. The product can have two, three or four meta bromine atoms, with three being preferred and four being most preferred.

The meta-brominated biphenols of this process can be incorporated into monomers and polymers as

are other biphenols, such as the meta-brominated biphenols described in US—A—3,943,191; 3,959,211; 3,974,235 and 3,989,531.

The invention is further illustrated by the following nonlimiting examples.

Example 1

Into a 3-necked flask were placed 24.3 g (0.10 mole) of 2,2',6,6'-tetramethylbiphenol and 100 ml (1.56 moles) of methylene chloride. The flask was cooled in a 20°C water bath. Then, 11.3 ml (0.22 mole) of bromine was added over a period of 5 minutes while the temperature of the reaction medium increased from 21°C to 27°C. This produced a slurry. The slurry was stirred for 30 minutes at a temperature of 25°C. The temperature of the reaction mixture was then increased to 40°C, and the mixture refluxed for 1 hour. The reaction mixture was then flash distilled under atmospheric conditions. The remainder of the solvent was removed using a rotary evaporator. This left 39 g of a dark solid which, when analyzed by gas chromatography, was revealed to be 87 mole percent dibromotetramethyl-biphenol, 8 mole percent monobromotetramethylbiphenol, 2 mole percent tribromotetramethylbiphenol and 3 mole percent unreacted tetramethylbiphenol. This is a conversion of 97 percent of the tetramethylbiphenol, and an isolated yield of 85 mole percent.

The dibromotetramethylbiphenol's identity was confirmed by comparison of nuclear magnetic resonance spectra of dibromotetramethylbiphenol prepared by another method.

Example 2

Example 1 was repeated except that 24.2 g (0.10 mole) of tetramethylbiphenol and 25.6 ml (0.50 mole) of bromine were employed. The reaction mixture was refluxed for 2 hours. A gas chromatographic analysis of this reaction product revealed 12 percent dibromotetramethylbiphenol, 81 mole percent tribromotetramethylbiphenol and 7 mole percent tetrabromotetramethylbiphenol. The reaction product was diluted with 150 ml of methylene chloride. The diluted reaction product was heated to a temperature of 40°C and the solvent was distilled until the distillate was clear. The resulting thick slurry was cooled to 25°C. The cooled slurry was filtered. The filtrate was air dried. A yield of 41.2 g of white solid product was obtained which had the following composition when analyzed by gas chromatography: 13 mole percent dibromotetramethylbiphenol, 84 mole percent tribromotetramethylbiphenol and 3 mole percent tetrabromotetramethylbiphenol. This corresponds to a 72 mole percent yield of tribromotetramethylbiphenol. Analysis of the solid by nuclear magnetic resonance showed no methyl brominated material.

Example 3

Example 1 was repeated except that 24.2 g (0.10 mole) of tetramethylbiphenol, 160 ml (2.50 moles) of methylene chloride, 16.4 ml (0.32 mole) of bromine and 0.4 g (0.0025 mole) of ferric chloride were employed. The reaction was refluxed for 1.5 hours at which time the refluxing liquid was clear indicating that the refluxing liquid contained no unreacted bromine. Half of the solvent was removed by flash distillation, and the rest was removed using a rotary evaporator. This left 49.3 g of solid, which when analyzed by gas chromatography had the following composition: 73 percent tribromotetramethylbiphenol, 3 percent tetramethylbiphenol, 10 percent dibromotetramethylbiphenol and 14 percent tetrabromotetramethylbiphenol. This corresponds to a 97 percent conversion and a 75 mole percent isolated yield of tribromotetramethylbiphenol.

Example 4

Example 1 was repeated except that 24.2 g (0.10 mole) of tetramethylbiphenol, 46.0 ml (0.90 mole) of bromine, and 100 ml (1.56 moles) of methylene chloride are employed. The reaction is refluxed at 40°C for 3 hours. The unreacted bromine was removed by distillation with the aid of an additional 200 ml of solvent. The slurry was cooled to 25°C and the rest of the solvent was removed in a rotary evaporator. Nuclear magnetic resonance analysis of the product did not detect any methyl bromination. Gas chromatographic analysis indicated 4 mole percent tribromotetramethylbiphenol and 96 mole percent tetrabromotetramethylbiphenol. Drying the solid at 110°C for 14 hours under vacuum yielded 55.7 g of a gray solid. This solid was dispersed in 70 ml of acetone and was refluxed for 1 hour. The slurry was then cooled, and the solid was filtered and dried under a vacuum at a temperature of 110°C for 4 hours. A white solid (51.0 g) was obtained. This solid had a purity of 98 mole percent as analyzed by gas chromatography. This solid has a melting point between 242°C and 245°C. This is an overall yield of 90 mole percent and overall conversion of 100 percent.

Example 4 demonstrates a selective, high yield process for the preparation of 3,3',5,5'-tetrabromo-2,2',6,6'-tetramethylbiphenol.

Example 5

Example 1 was repeated except that 24.2 g (0.10 mole) of tetramethylbiphenol, 21.5 ml (0.42 mole) of bromine, 150 ml (2.34 moles) of methylene chloride, and 0.4 g (0.0025 mole) of ferric chloride were employed. The reaction was refluxed for 2 hours. Solvent (100 ml) was removed, and the slurry was cooled to 25°C. The insoluble solid was filtered, rinsed with 50 ml of solvent, and dried under vacuum at 70°C for 14

hours. A yield of 52.5 g of light brown solid was obtained. Gas chromatographic analysis indicated 4 mole percent tribromotetramethyl-biphenol and 96 mole percent tetrabromotetramethylbiphenol. Nuclear magnetic resonance analysis of the product did not detect any methyl bromination. This corresponds to a 90 mole percent yield of tetrabromotetramethylbiphenol.

Example 5 demonstrates a selective, high yield catalytic process for the preparation of 3,3',5,5'-tetra-bromo-2,2',6,6'-tetrabromomethylbiphenol.

**Claims**

1. A process for preparing a meta-brominated biphenol having at least 2 meta-bromine atoms and having the formula

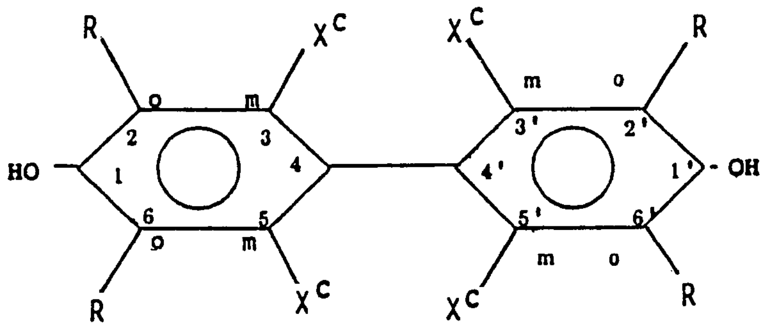

wherein R is a$C_1$—$C_8$ alkyl or alkoxy group, X is Br or hydrogen characterized in that a starting compound of the formula

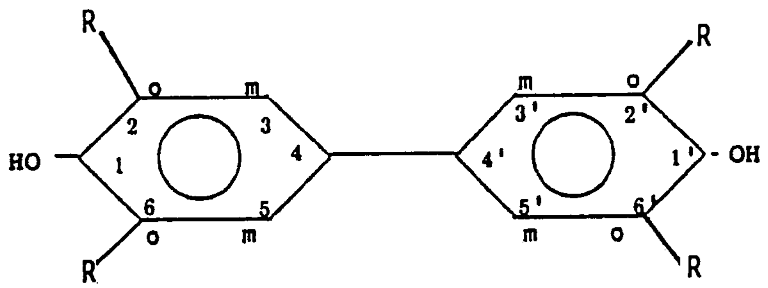

having all its ortho positions blocked with a $C_1$—$C_8$ alkyl or alkoxy group R, is contacted with a brominating agent selected from bromine, bromine chloride and hypobromites at a temperature range from 0 to 100°C.

2. The process of claim 1 wherein the reaction is conducted neat.

3. The process of claim 1 wherein a reaction medium is present.

4. The process of claim 3 wherein the reaction medium is methylene chloride.

5. The process of claim 1 wherein a catalyst is present.

6. The process of claim 5 wherein the catalyst is aluminum bromide, aluminum chloride or ferric chloride.

**Patentansprüche**

1. Verfahren zum Herstellen eines meta-bromierten Biphenols mit mindestens 2 Bromatomen in meta-Stellung der Formel

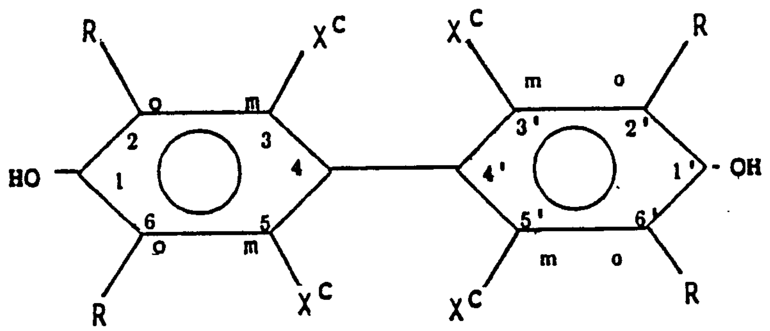

in der R eine $C_1$—$C_8$ Alkyl- oder Alkoxygruppe ist, X ist Brom oder Wasserstoff, dadurch gekennzeichnet, daß eine Ausgangsverbindung der Formel

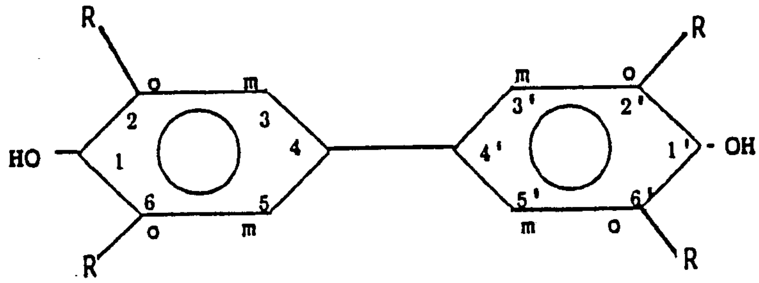

bei der alle Orthostellungen mit einer $C_1$—$C_8$ Alkyl- oder Alkoxygruppe R blockiert sind, mit einem aus Brom, Chlorbrom und Hypobromiten ausgewählten Bromierungsmittel bei einer Temperatur im Bereich von 0 bis 100°C in Berührung gebracht wird.

2. Verfahren nach Anspruch 1, dadurch gekennzeichnet, daß die Reaktion unverdünnt ausgeführt wird.

3. Verfahren nach Anspruch 1, dadurch gekennzeichnet, daß ein Reaktionsmedium vorhanden ist.

4. Verfahren nach Anspruch 3, dadurch gekennzeichnet, daß das Reaktionsmedium Methylenchlorid ist.

5. Verfahren nach Anspruch 1, dadurch gekennzeichnet, daß ein Katalysator vorhanden ist.

6. Verfahren nach Anspruch 5, dadurch gekennzeichnet, daß der Katalysator Aluminiumbromid, Aluminiumchlorid oder Eisen-(III)-chlorid ist.

**Revendications**

1. Procédé de préparation d'un bisphénol bromé en méta comprenant au moins 2 atomes de brome en position méta et de formule

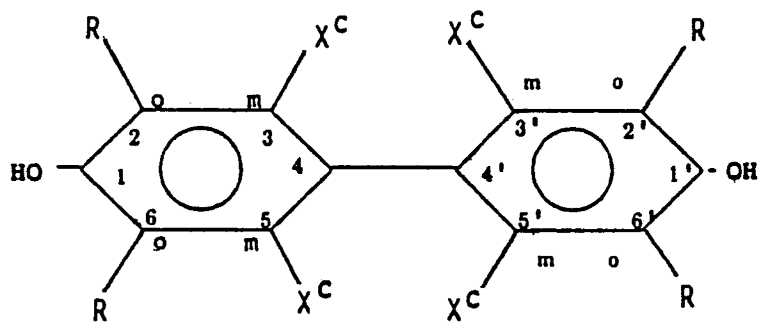

dans laquelle R représente un groupe alkyle ou alcoxy en $C_1$ à $C_8$, X est Br ou H, caractérisé en ce que l'on fait réagir un composé de départ de formule

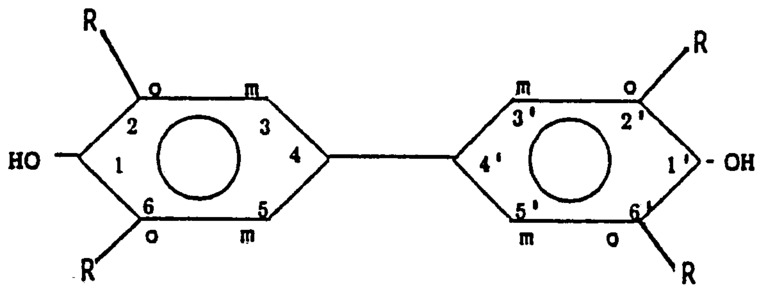

ayant toutes ses positions ortho bloquées par un groupe R alkyle ou alcoxy en $C_1$ à $C_8$ avec un agent de bromation choisi dans le groupe constitué par le brome, le chlorure de brome et les hypobromites, à une température comprise entre 0 et 100°C.

2. Procédé de la revendication 1, dans lequel la réaction est effectuée telle quelle.

3. Procédé de la revendication 1, dans lequel un milieu réactionnel est présent.

4. Procédé de la revendication 3, dans lequel le milieu réactionnel est le chlorure de méthylène.

5. Procédé de la revendication 1, dans lequel un catalyseur est présent.

6. Procédé de la revendication 5, dans lequel le catalyseur est le bromure d'aluminium, le chlorure d'aluminium ou le chlorure ferrique.